Europäisches Patentamt

European Patent Office

Office européen des brevets

(19)

(11) Publication number: **0 105 587**
**B1**

(12) **EUROPEAN PATENT SPECIFICATION**

(45) Date of publication of patent specification: **12.08.87**

(21) Application number: **83304692.3**

(22) Date of filing: **15.08.83**

(51) Int. Cl.⁴: **A 61 M 1/14,** B 29 C 65/30,
F 16 L 47/02

(54) Automatic splicing device.

(30) Priority: **16.08.82 US 408418**

(43) Date of publication of application:
**18.04.84 Bulletin 84/16**

(45) Publication of the grant of the patent:
**12.08.87 Bulletin 87/33**

(84) Designated Contracting States:
**BE CH DE FR GB IT LI NL SE**

(56) References cited:
**EP-A-0 044 204**
**US-A-3 897 296**
**US-A-4 022 256**

(73) Proprietor: **E.I. DU PONT DE NEMOURS AND
COMPANY
1007 Market Street
Wilmington Delaware 19898 (US)**

(72) Inventor: **Benin, Joshua
13 Cauline Court
Newark Delaware 19711 (US)**
Inventor: **Krauss, Leland Luper
63 Hidden Valley Road
Aston Pennsylvania 19014 (US)**
Inventor: **Roxlo, Helen Elizabeth
225 West Park Place
Newark Delaware 19711 (US)**

(74) Representative: **Jones, Alan John et al
CARPMAELS & RANSFORD 43 Bloomsbury
Square
London, WC1A 2RA (GB)**

Courier Press, Leamington Spa, England.

## Description

This invention relates to an apparatus and process for forming a sterile connection between two plastic tubes.

At the present time there are a number of medical and scientific procedures which require the sterile transfer of fluids from one container to another. Two areas in which sterile splicing of tubes would be useful are in replacing dialysate liquid bags in continuous ambulatory peritoneal dialysis (CAPD) procedures, and in processing blood in bags to separate components, add treating agents, etc.

EP—A—0 044 204 discloses a process of forming a sterile connection between a first tube formed of a thermoplastic resin and a second tube formed of a thermoplastic resin comprising mounting said tubes in a pair of mounting means which hold said tubes in a closely adjacent substantially parallel position, urging an edge of a hot cutting means having substantially flat surfaces through both said tubes at a rate such that the thermoplastic resin from which said tubes are formed which is in contact with said cutting means is molten, whereby a molten interface is formed between the substantially flat surfaces of said cutting means and each of said tubes, maintaining said molten interfaces while aligning said tubes with each other and then passing said tubes over an edge of said cutting means while urging said tubes together, and permitting the thusly joined molten interfaces to cool whereby a sterile connection is formed between said tubes.

EP—A—0 044 204 further discloses an apparatus for forming a sterile connection comprising a cutting means; means adapted to heat said cutting means; a pair of mounting blocks adapted to receive and hold two tubes to be joined; means for movement of the mounting blocks to first, second and third positions, the cutting means being between the mounting blocks in the first position, said mounting blocks being relatively displaced in said second position to align two different tube ends facing each other, said mounting blocks being separated from said cutting means in said third position, and spring means urging said mounting blocks together when in the third position.

A splicing device which would automatically produce sterile splices between two internally sterile tubes in a simple, rapid, reliable manner would permit one to effect whatever processing is desired in such areas without compromising sterility.

The present invention provides an apparatus for forming a connection between two thermoplastic tubes comprising: a pair of displaced mounting blocks for holding said tubes in a side-by-side relationship; a heated cutting means located between the mounting blocks, said mounting blocks being movable with respect to each other and with respect to the cutting means; means for providing movement between said mounting blocks and said cutting means, said means for providing movement generating three pivoting orthogonal motions; and a controller coupled to the means for providing movement to control the timing of the operation of the apparatus.

The present invention further provides a process for forming a connection between two thermoplastic tubes being held in a side-by-side relationship in a pair of displaced mounting blocks movable relative to each other and movable with respect to a heated cutting means between the mounting blocks comprising: passing the cutting means through the tubes; shifting the mounting blocks to align two different cut tube ends facing each other while maintaining contact between the cut tube ends and the cutting means; and withdrawing the cutting means from contact with the tubes while urging the mounting blocks together, said passing, shifting and urging steps being pivotal orthogonal motions; and controlling the timing of said process to automatically obtain said connection.

The present invention particularly relates to an apparatus and process for cutting and joining two internally sterile tubes using a heated cutting means while maintaining system sterility. The tubes to be joined are cut by the cutting means with molten polymer sealing each tube end against the cutting means. Seals of molten polymer hot enough to kill bacteria quickly are effectively maintained with no chance for viable airborne or surface bacteria to find their way inside either of the tubes or the joint. The tube ends are moved into alignment, the heated cutting means slid away, and the ends pushed together. When the joint cools, the sterile connection is complete.

The apparatus includes, in addition to the heated cutting means, a pair of mounting blocks adapted to receive and hold the tubes and a family of mechanisms to generate three pivoting orthogonal motions required in splicing. These are moving the hot blade and the tubes relative to each other to pass the blade through the tubes, shifting the mounting blocks or clamps holding the tubes relative to each other to align the tube ends to be joined, and finally as the hot blade is moved from contact with the tubes, pushing or urging the mounting blocks together. The apparatus also includes a controller to provide precise timing of the events outlined above for effecting a sterile connection and to provide safeguards to insure sterility is maintained.

Brief description of the drawings

Fig. 1 is an isometric view of the automatic splicing device of this invention.

Fig. 1A is an isometric view of the blade holder and load and eject device used in the splicing device of this invention.

Fig. 2 is an isometric view partially broken away of the driven cam used to move the clamps and the blade of the splicing device of Fig. 1.

Fig. 3 is a view of Fig. 2 taken along line 3—3.

Fig. 4 is a view of Fig. 2 taken along line 4—4.

Figs. 5A—5D are schematic views of two tubes being positioned, severed, realigned and joined, respectively.

Fig. 6 is an isometric view of the mounting blocks of the splicing device of this invention.

Fig. 7 is a block diagram of the control system for the splicing device of this invention.

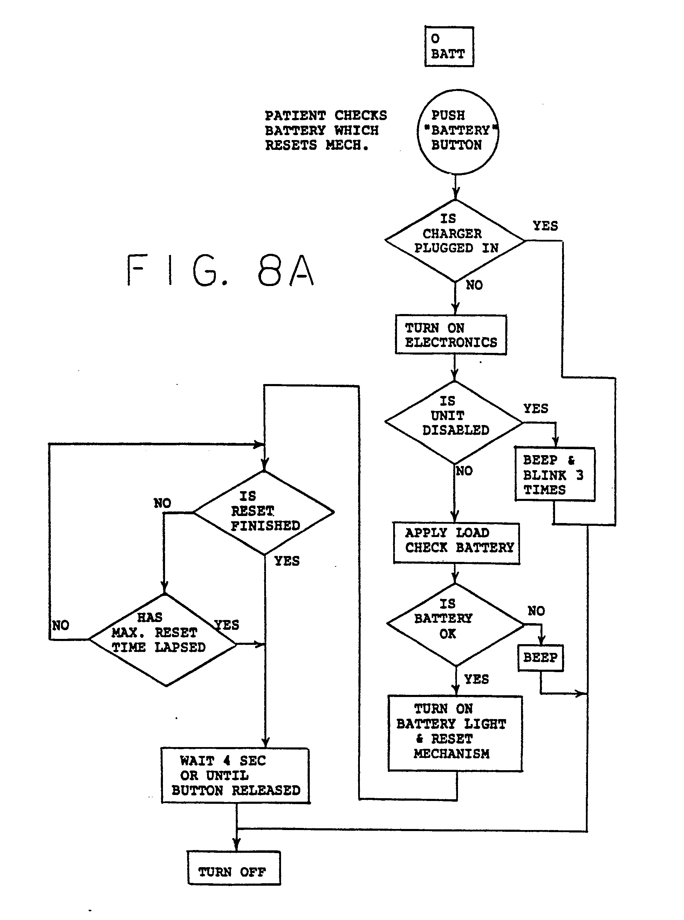

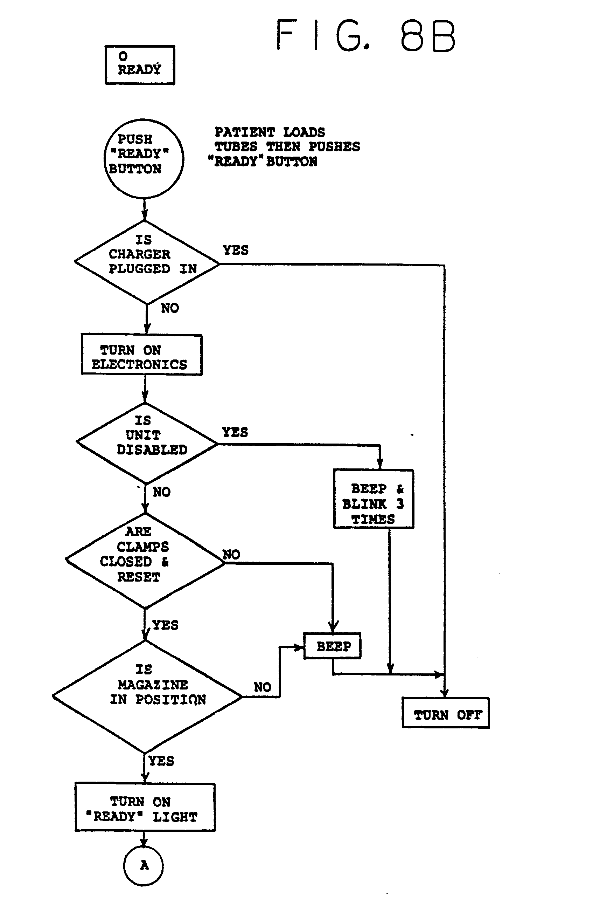

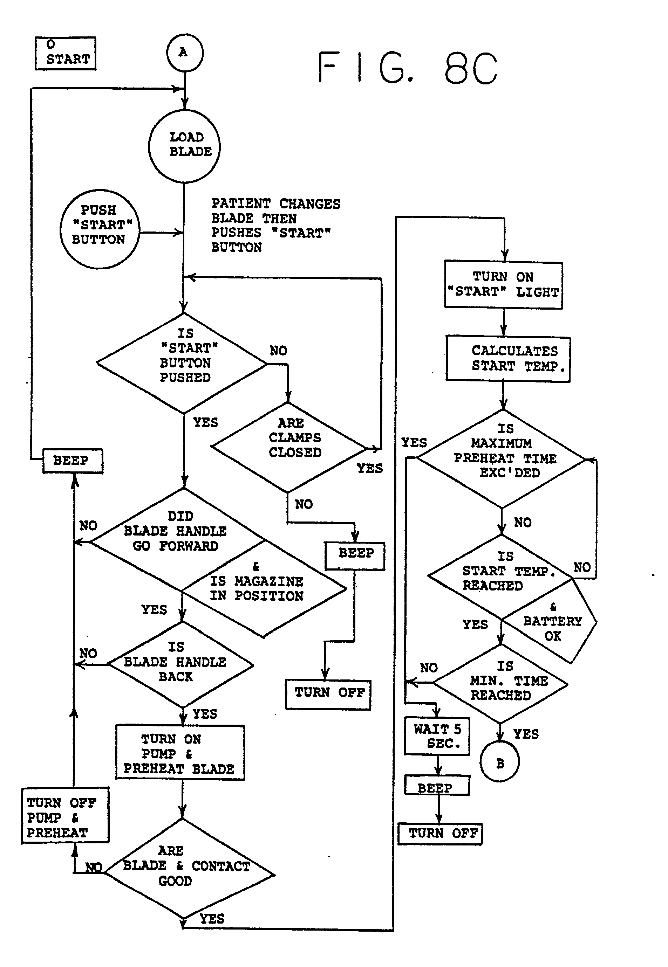

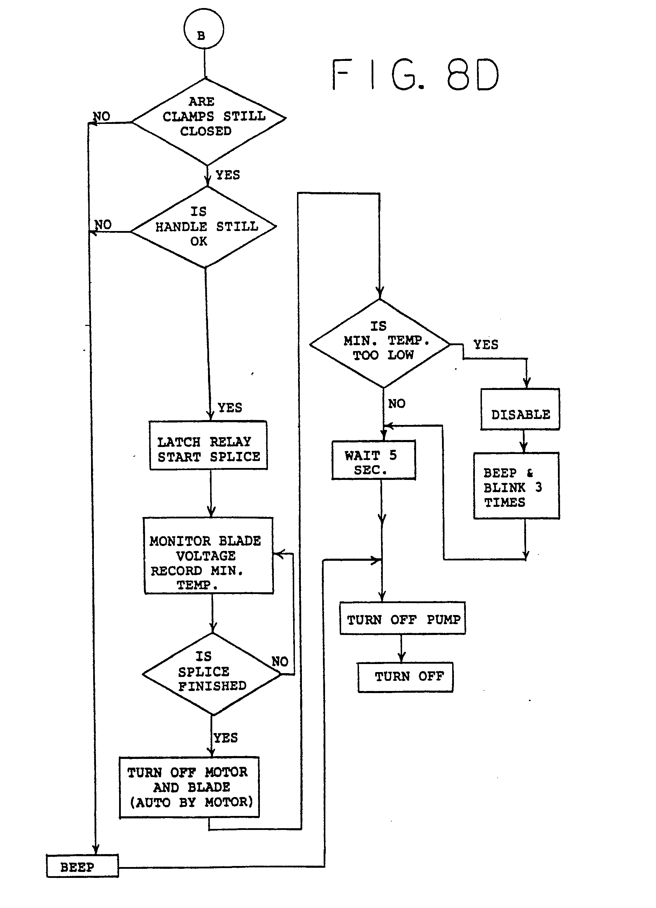

Figs. 8A—8D are logic diagrams for the splicing device of this invention.

Detailed description of the invention

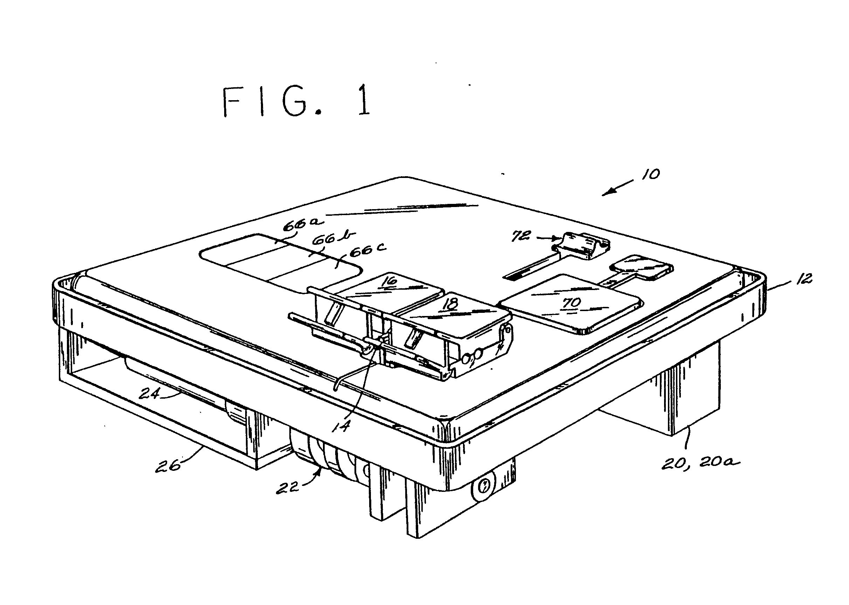

Referring to Fig. 1, the splicing device chosen for purposes of illustration is denoted generally as 10 and includes as major components a frame 12, a cutter 14 pivotally connected to the frame, a pair of mounting blocks 16, 18 spaced from each other in the same plane, an evacuation pump 20a driven by a motor 20, a cam cylinder 22 driven by a motor 24 and the electronic control unit 26.

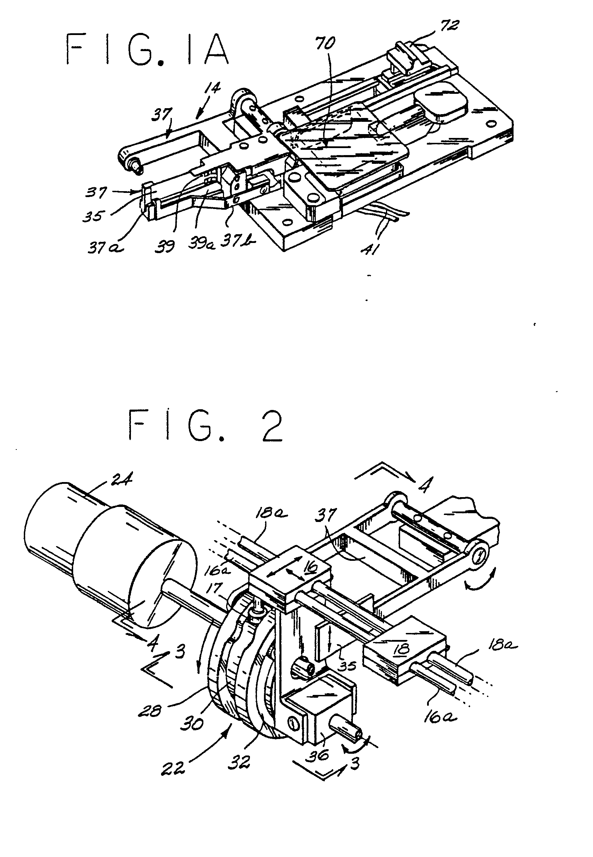

The mechanism for generating the three orthogonal motions required for splicing is seen in Fig. 2 which shows a typical arrangement of that mechanism. More particularly, it involves three cams to accomplish the three motions. In the preferred embodiment shown, the cams are grooves 28, 30, 32 on different faces of cam cylinder 22. This arrangement ensures that the three cams never get out of phase. A knife holder 37 for blade 35 is pivotally attached to frame 12 at one end and is engaged in cam groove 28 at its other end. The blade 35 is positioned between mounting blocks 16, 18 and below the tubes 16a and 18a held side-by-side in the blocks for splicing. A pivoting block 36 is journaled in frame 12 at one end and journaled to mounting block 16 at its other end. Mounting block 16 intermediate to its ends is engaged in cam groove 32. Mounting block 16 is also engaged in peripheral cam groove 30 via follower 17 while mounting block 18 is fixed to frame 12. Motor 24 rotates cam cylinder 22.

The blade 35 for the cutting knife is an etched stainless steel foil resistor laminated between sheets of copper connected to a battery. In use, it is subjected to a short heating cycle (about 6 seconds) and to one-shot use (a new blade is used for each splice).

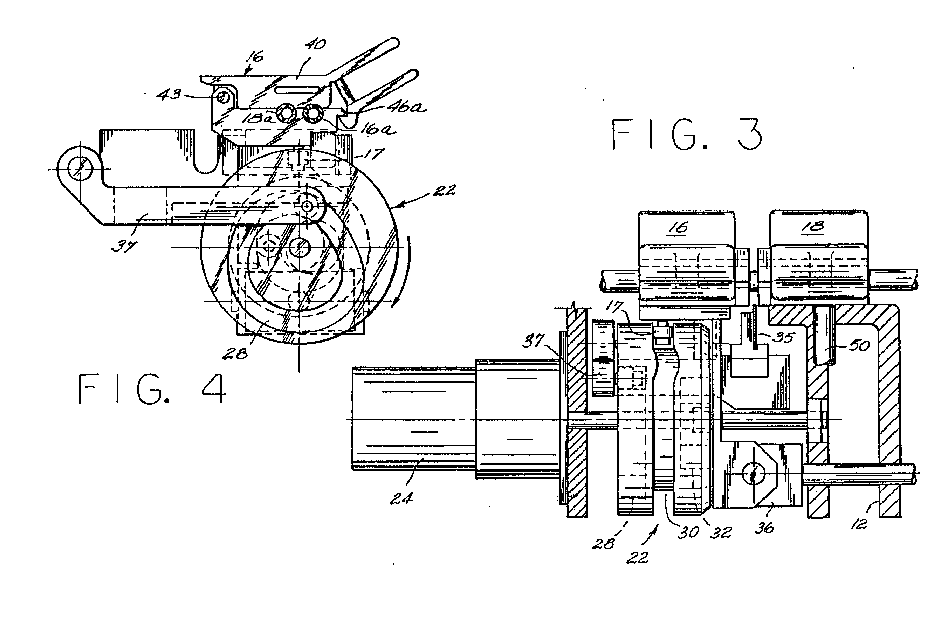

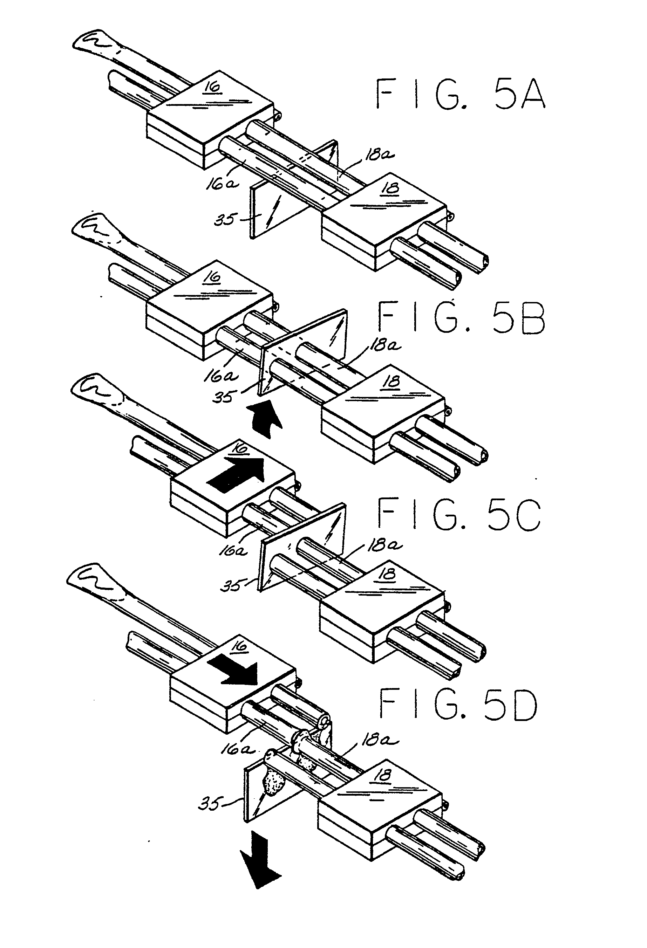

The splicing operation with the apparatus disclosed utilizes three orthogonal motions involving mounting block 16 and the knife blade 35. These are lifting the knife blade 35 through the tubes 16a and 18a, shifting the tubes to align the ones to be joined together and finally urging the tubes together while withdrawing the blade. Figs. 5A—5D schematically shows this operation and in 5A the mounting blocks 16, 18 and knife blade 35 are shown in the ready position after the blade is heated to operating temperature. The cam cylinder 22 commences rotation (Fig. 4) in the direction of the arrow and with this rotation cam groove 28 lifts knife blade 35 upwardly through the tubes 16a, 18a. With the blade 35 dwelling between the tubes, continued rotation of the cam cylinder causes cam groove 32 to rotate mounting block 16 aligning the tubes 16a, 18a (Fig. 5C).

Continued rotation of the cam cylinder causes peripheral cam groove 30 to urge mounting block 16 toward fixed mounting block 18 as knife blade 35 is lowered. Thus tubes 16a, 18a are pushed together forming a sterile splice between them (5D).

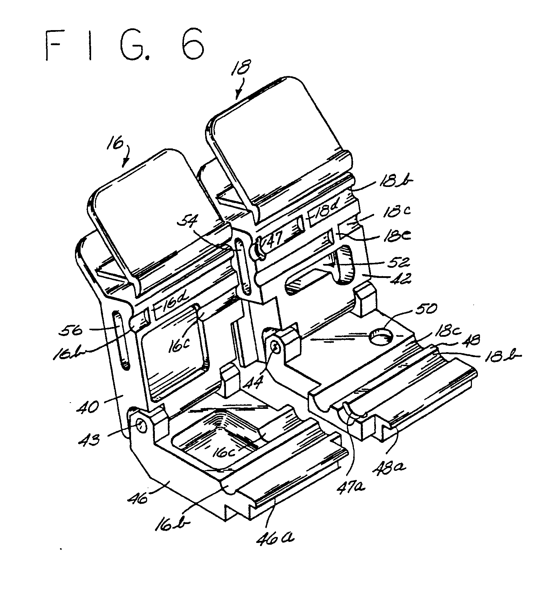

As best shown in Fig. 6, the tube mounting blocks 16, 18 include covers 40, 42 pivotally attached at hinge points 43, 44 to tube holder bases 46, 48. The fixed mounting block 18 has a relief groove 47 cut in its cover 42 with a matching groove 47a in its holder 48 to accommodate an old splice. Channels 16b, 16c are provided in mounting block 16 to hold the tubes to be spliced and mounting block 18 is similarly equipped with channels 18b, 18c. Mounting block 16 is also shown with a closed end to channel 16c to position the tube and permit finger access for removal of the severed off end of tube 18a. The channel 16b in cover 40 is provided with a web 16d and the channels 18b and 18c in cover 42 are provided with webs 18d and 18e for the purpose of clamping the tubes positively in place when the covers are latched in place over the lips 46a and 48a of the respective bases 46, 48. Also incorporated in the mounting block 18 is a passage 50 in the base 48 that is connected to evacuation pump 20a. Passage 50 is in communication with recess 52 in cover 42 and intersecting channel 54 when mounting block 18 is closed to pull air above the tubes as they are being melted by the hot knife 35. The fumes from the splicing operation are drawn off and filtered in a filter (not shown) attached to the inlet of pump 20a. The mounting block 16 also has a channel 56 in its cover 40 to provide a direct path for air to be drawn above the tubes into the cover 42.

The knife blade load and eject system is best seen in Fig. 1A and includes a blade magazine 70, a blade load-eject pusher lever 72, blade holder 37, blade clamp 37b and a track 37a in the blade holder. A pair of spring loaded electrical contacts 39, 39a joined by leads 41 to controller 26 are making contact with the blade 35.

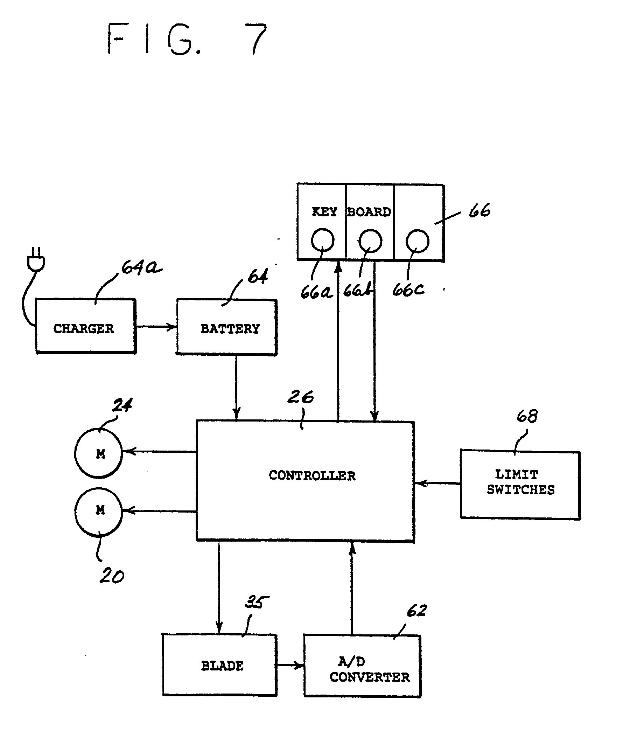

Fig. 7 is a block diagram of the control system of the splicing device described above and includes a controller 26 coupled to a keyboard 66 which includes push buttons 66a, 66b, 66c labelled Battery, Ready and Start, respectively. A battery 64 and its associated charger 64a as well as certain limit switches designated generally as 68 and an analog to digital converter 62 provide inputs to the controller which in turn controls the operation of the heating of knife blade 35 and the operation of cam motor 24 and evacuation pump motor 20. The controller 26 comprises an 8-bit microprocessor No. ID 8085AH, a programmable read only memory No. ID 8755A and a programmable random access memory No. ID 8155H, all by Intel. The A/D converter 62 is a No. ADC 0802LD by National Semiconductor.

With reference to the logic flow diagrams 8A—8D the operation of the automatic splicing device of this invention will be described as it would be used by a CAPD patient.

First of all, it should be noted that to insure patient safety from shock in CAPD applications, the unit is powered by a 24 volt DC rechargeable battery 64 and control circuitry is included that turns off the device in the cycle if the charger 64a is plugged in.

Initially the patient does not have any tubes in the mounting blocks 16, 18 and the unit must be reset from the last splice cycle and the battery condition checked. The patient pushes the "BATTERY" button 66a which causes the control system to check that the battery has an acceptable charge and turn on the motor 24 to rotate a cam cylinder 22 which resets the moveable clamp 16 to the start position and actuates the "home" position switch (not shown). The patient then places the tubes to be spliced in the mounting blocks 16, 18 and latches the covers 40, 42 which actuates switches (not shown) that indicate the mounting blocks are closed.

The patient pushes the "READY" button 66b which causes the control system to turn on the electronics, check that a blade magazine is installed, and check that the mounting block covers are closed.

The patient pushes the blade load/eject lever 72 forward which cams open the electrical 39, 39a contacts to the blade 35 and opens the blade clamp 37b, pushes against a blade 35 from the blade magazine 70 which then pushes against other blades in the blade holder to cause the previously used blade 35 to be ejected and a fresh blade to be positioned for the next splice, (NOTE: If the blade magazine is empty, the blade pusher in the magazine mechanically blocks the blade loader/ejector from completing its forward stroke) and actuates a forward switch (not shown) that indicates a fresh blade is properly positioned for the next splice.

The patient retracts the blade load/eject lever 72 which releases the blade clamp 37b to clamp the blade, releases the electrical contacts 39, 39a to press against the blade contact surfaces, releases the next blade in the blade magazine to position it for the next load/eject cycle and actuates a retract switch (not shown) that indicates the lever is retracted and the electrical contacts and blade clamp should be engaged.

The patient pushes the "START" button 66c which causes the control system to turn on the fume evacuation pump 20, check that the blade load/eject lever 72 went forward and back and a magazine 70 was in position, recheck that the tube clamp covers are latched, turn on constant current to the blade and check the voltage after 0.5 seconds to determine if the blade contact and integrity are good. If the above are OK, the control system continues applying constant current to the blade to preheat it for the splice and monitors voltage and time during the preheat cycle to verify the blade integrity and determine when the temperature is right for making a sterile splice. When the proper temperature is reached for the splice, the control system checks that the clamp covers are still closed and the blade load/eject lever is retracted and actuates the splice relay (not shown) that provides a latched electrical circuit for the blade current and motor 24 so the splice can be completed independent of the microprocessor. This latched circuit insures high reliability for the actual splice operation when the sterility of the tubing is being threatened.

The splice motor 24 rotates the cam cylinder 22 which completes a splice and actuates a switch (not shown) that unlatches the splice relay which turns off the motor 24 and blade current.

The control system then turns off the fume evacuation pump motor 20 and checks the minimum voltage monitored during the splicing operation to ensure the temperature remained high enough to guarantee the sterility of the splice and activates an alarm if it isn't.

**Claims**

1. An apparatus (10) for forming a connection between two thermoplastic tubes (16a, 18a) comprising: a pair of displaced mounting blocks (16, 18) for holding said tubes (16a, 18a) in a side-by-side relationship; a heated cutting means (35) located between the mounting blocks (16, 18), said mounting blocks being movable with respect to each other and with respect to the cutting means (35), means (22, 24) for providing movement between said mounting blocks and said cutting means, said means (22, 24) for providing movement generating three pivoting orthogonal motions; and a controller (26) coupled to the means (22, 24) for providing movement to control the timing of the operation of the apparatus (10).

2. The apparatus as defined in claim 1, said means (22, 24) for providing movement being a driven cam cylinder (22) containing one groove (28, 32) in each face and one groove (30) around its periphery, one (16) of said mounting blocks being coupled to the groove (32) in one face of said cam (22) and to the groove (30) around the periphery of the cam (22), said cutting means (35) being coupled to the other face of said cam (22).

3. The apparatus as defined in claim 1 or 2, including an evacuation pump (20), said mounting blocks (16, 18) having a passage (50) in communication with a separation between said mounting blocks (16, 18) above said tubes (16a, 18a) at its one end and said passage (50) being in communication with said pump (20) at its other end.

4. The apparatus as defined in any one of Claims 1 to 3, said mounting blocks (16, 18) being movable relative to each other to first, second, and third positions, the cutting means (35) being between the mounting blocks (16, 18) in the first position, said mounting blocks (16, 18) being relatively displaced in said second position to align two different tube ends facing each other, said mounting blocks (16, 18) being separated from said cutting means (35) in said third position while said mounting blocks (16, 18) are urged together; the movement between the heated cutting means (35) and both of said mounting blocks (16, 18) passing the cutting means (35) through said tubes (16a, 18a) when the mounting blocks (16, 18) are in said first position and withdrawing said cutting means (35) from the contact with said tubes (16a, 18a) when said mounting blocks (16, 18) are in said third position.

5. Apparatus as defined in Claim 4 further comprising: a frame (12); said cutting means (35) being pivotally mounted to said frame (12); means (39) for heating said cutting means (35); means (22, 24) for moving said mounting blocks (16, 18) to said first, second, and third positions, means (22, 24) for moving the heated cutting means (35) through both of said tubes (16a, 18a) when the mounting blocks (16, 18) are in said first position and withdrawing said cutting means (35) from the contact with said tubes (16a, 18a) when said mounting blocks (16, 18) are in said third position; and said controller (26) being coupled to the means (39) for heating the cutting means (35), the means (22, 24) for moving the mounting blocks (16, 18) and the means (22, 24) for moving the cutting means to precisely control the timing of the operation of said apparatus to automatically obtain a connection between the tubes (16a, 18a).

6. Apparatus as defined in Claim 5 wherein one (18) of said mounting blocks is fixed to said frame (12), the other (16) being movable.

7. A process for forming a connection between two thermoplastic tubes (16a, 18a) being held in a side-by-side relationship in a pair of displaced mounting blocks (16, 18) movable relative to each other and movable with respect to a heated cutting means (35) between the mounting blocks (16, 18) comprising: passing the cutting means (35) through the tubes (16a, 18a); shifting the mounting blocks (16, 18) to align two different cut tube ends facing each other while maintaining contact between the cut tube ends and the cutting means (35); and withdrawing the cutting means (35) from contact with the tubes (16a, 18a) while urging the mounting blocks (16, 18) together, said passing, shifting and urging steps being pivotal orthogonal motions; and controlling the timing of said process to automatically obtain said connection.

**Patentansprüche**

1. Vorrichtung (10) zur Verbindung zweier thermoplastischer Schläuche (16a, 18a), bestehend aus: zwei im Abstand angeordneten Halteblöcken (16, 18), welche die Schläuche (16a, 18a) nebeneinander halten; einer zwischen den Halteblöcken (16, 18) angeordneten erwärmbaren Schneidvorrichtung (35), wobei die Halteblöcke zueinander und im Verhältnis zur Schneidvorrichtung (35) bewegbar sind; einer Vorrichtung (22, 24) zum Erzeugen einer Bewegung zwischen den Halteblöcken und der Schneidvorrichtung, wobei die Bewegungsvorrichtung (22, 24) drei rechtwinklige Drehbewegungen erzeugt; und einer mit der Bewegungsvorrichtung (22, 24) verbundenen Steuervorrichtung (26) zum Steuern des zeitlichen Verfahrensablaufs der Vorrichtung (10).

2. Vorrichtung nach Anspruch 1, bei der die Bewegungsvorrichtung (22, 24) ein angetriebener Kurvenzylinder (22) ist, der eine Nut (28, 32) in jeder Stirnfläche und eine Umfangsnut (30) aufweist, wobei einer (16) der Halteblöcke mit der Nut (32) in einer Stirnfläche der Nocke (22) und mit der Umfangsnut (30) der Nocke (22) gekuppelt ist, und die Schneidvorrichtung (35) mit der anderen Stirnfläche der Nocke (22) gekuppelt ist.

3. Vorrichtung nach Anspruch 1 oder 2, mit einer Saugpumpe (20), wobei die Halteblöcke (16, 18) einen Durchlaß (50) aufweisen, der an seinem einen Ende mit einer Abteilung zwischen den Halteblöcken (16, 18) oberhalb der Schläuche (16a, 18a) verbunden ist und welcher an seinem anderen Ende an die Pumpe (20) angeschlossen ist.

4. Vorrichtung nach einem der Ansprüche 1 bis 3, bei der die Halteblöcke (16, 18) relativ zueinander in eine erste, zweite und dritte Position bewegbar sind, wobei sich in der ersten Position die Schneidvorrichtung (35) zwischen den Halteblöcken (16, 18) befindet, in der zweiten Position die Halteblöcke (16, 18) relativ zueinander verschoben sind, um zwei einander gegenüberliegende verschiedene Schlauchenden fluchtend auszurichten, und in der dritten Position die Halteblöcke (16, 18) gegeneinander gedrückt und gleichzeitig von der Schneidvorrichtung (35) getrennt sind; und bei der die Bewegung der erwärmten Schneidvorrichtung (35) und der beiden Halteblöcke (16, 18) zueinander die Schneidvorrichtung (35) durch die Schläuche (16a, 18a) führt, wenn die Halteblöcke (16, 18) sich in der ersten Position befinden, und die Schneidvorrichtung (35) aus der Berührung mit den Schläuchen (16a, 18a) zurückzieht, wenn sich die Halteblöcke (16, 18) in der dritten Position befinden.

5. Vorrichtung nach Anspruch 4, weiter bestehend aus: einem Rahmen (12), an welchem die Schneidvorrichtung (35) drehbar montiert ist; einer Vorrichtung (39) zum Erwärmen der Schneidvorrichtung (35); einer Vorrichtung (22, 24), die die Halteblöcke (16, 18) in die erste, zweite und dritte Position bewegt, und die die erwärmte Schneidvorrichtung (35) durch beide Schläuche (16a, 18a) bewegt, wenn sich die Halteblöcke (16, 18) in der ersten Position befinden, und die die Schneidvorrichtung (35) aus der Berührung mit den Schläuchen (16a, 18a) zurückzieht, wenn sich die Halteblöcke (16, 18) in der dritten Position befinden; wobei die Steuervorrichtung (26) mit der Vorrichtung (39) zum Erwärmen der Schneidvorrichtung (35), der Vorrichtung (22, 24) zum Bewegen der Halteblöcke (16, 18) und der Vorrichtung (22, 24) zum Bewegen der Schneidvorrichtung gekoppelt ist, um den Arbeitsablauf der Vorrichtung zeitlich präzise zu steuern, so daß die Schläuche (16a, 18a) automatisch verbunden werden.

6. Vorrichtung nach Anspruch 5, bei der einer (18) der Halteblöcke an dem Rahmen (12) befestigt und der andere (16) bewegbar ist.

7. Verfahren zur Verbindung von zwei thermoplastischen Schläuchen (16a, 18a), die nebeneinander in zwei verschobenen Halteblöcken (16, 18) gehalten sind, welche relativ zueinander und relativ zu einer zwischen den Halteblöcken (16, 18) angeordneten erwärmten Schneidvorrichtung (35) bewegbar sind, bei dem man die Schneidvorrichtung (35) durch die Schläuche (16a, 18a) hindurchführt; die Halteblöcke (16, 18) verschiebt, um zwei verschiedene, sich gegenüberliegende abgeschnittene Schlauchenden fluchtend auszurichten, während man die Berührung zwischen den abgeschnittenen Schlauchenden und der Schneidvorrichtung (35) aufrechterhält; die Schneidvorrichtung (35) aus der Berührung mit den Schläuchen (16a, 18a) zurückzieht, während man die Halteblöcke (16, 18) gegeneinander drückt, wobei die Schritte des Durchführens, des Verschiebens und des Gegeneinanderdrückens als rechtwinklige Schwenkbewegungen erfolgen; und den zeitlichen Verfahrensablauf so steuert, daß die Verbindung automatisch hergestellt wird.

**Revendications**

1. Appareil (10) pour former une jonction entre deux tubes (16a, 18a) en matière thermoplastique, comprenant: deux blocs de fixation décalés (16, 18) pour maintenir les tubes (16a, 18a) dans une disposition relative côte à côte, un organe de coupe (35) chauffé disposé entre les blocs de fixation (16, 18), ces blocs étant mobiles l'un par rapport à l'autre et par rapport à l'organe de coupe (35), des moyens (22, 24) pour assurer un déplacement entre lesdits blocs de fixation et ledit organe de coupe, lesdits moyens (22, 24) pour assurer le déplacement engendrant trois mouvements orthogonaux de pivotement, et un régulateur (26) couplé aux moyens (22, 24) pour assurer un déplacement afin de commander le minutage du fonctionnement de l'appareil (10).

2. Appareil suivant la revendication 1, lesdits moyens (22, 24) pour assurer un déplacement étant un cylindre mené (22) formant came comportant une rainure (28, 32) dans chaque face et une rainure (30) autour de sa périphérie, l'un (16) desdits blocs de fixation étant couplé à la rainure (32) dans une face de ladite came (22) et à la rainure (30) autour de la périphérie de la came (22), ledit organe du coupe (35) étant couplé à l'autre face de ladite came (22).

3. Appareil suivant la revendication 1 ou 2, comprenant une pompe (20) d'aspiration, lesdits blocs de fixation (16, 18) comportant un passage (50) communiquant avec une séparation entre lesdits blocs de fixation (16, 18) au-dessus desdits tubes (16a, 18a) à sa première extrémité et ledit passage (50) communiquant avec ladite pompe (20) par son autre extrémité.

4. Appareil suivant l'une quelconque des revendications 1 à 3, lesdits blocs de fixation (16, 18) étant mobiles l'un par rapport à l'autre vers une première, une seconde et une troisième positions, l'organe de coupe (35) étant entre les blocs de fixation (16, 18) dans la première position, ces blocs (16, 18) étant relativement déplacés dans ladite seconde position pour aligner deux extrémités différentes de tubes se faisant face, lesdits blocs (16, 18) de fixation étant espacés dudit organe de coupe (35) dans ladite troisième position tandis que ces blocs sont forcés l'un vers l'autre, le déplacement entre ledit organe de coupe chauffé (35) et les deux blocs de fixation (16, 18) faisant passer l'organe de coupe (35) à travers les tubes (16a, 18a) lorsque les blocs de fixation (16, 18) sont dans ladite première position et retirant ledit organe de coupe (35) du contact avec lesdits tubes (16a, 18a) lorsque les blocs de fixation (16, 18) sont dans ladite troisième position.

5. Appareil suivant la revendication 4, comprenant en outre: un bâti (12), ledit organe de coupe (35) étant articulé sur ledit bâti (12), des moyens (39) pour chauffer ledit organe de coupe (35), des moyens (22, 24) pour déplacer lesdits blocs de fixation (16, 18) dans lesdites première, seconde et troisième positions, des moyens (22, 24) pour déplacer l'organe de coupe (35) chauffé à travers les deux tubes (16a, 18a) lorsque les blocs de fixation (16, 18) se trouvent dans ladite première position et pour retirer ledit organe de coupe (35) du contact avec les tubes (16a, 18a) lorsque lesdits blocs de fixation (16, 18) sont dans ladite troisième position, et ledit régulateur (26) étant couplé aux moyens (39) pour chauffer l'organe de coupe (35), aux moyens (22, 24) pour déplacer les blocs de fixation (16, 18) et aux moyens (22, 24) pour déplacer l'organe de coupe afin de commander avec précision le minutage du fonctionnement dudit appareil et obtenir automatiquement une jonction entre les tubes (16a, 18a).

6. Appareil suivant la revendication 5, dans lequel l'un desdits blocs de fixation est fixé sur ledit bâti (12), l'autre (16) étant mobile.

7. Procédé pour former une jonction entre deux tubes thermoplastiques (16a, 18a) maintenus dans une disposition relative côte à côte dans deux blocs de fixation décalés (16, 18) mobiles l'un par rapport à l'autre et mobiles par rapport à un organe de coupe chauffé (35) entre les blocs de fixation (16, 18), suivant lequel: on fait passer l'organe de coupe (35) à travers les tubes (16a, 18a), on déplace les blocs de fixation (16, 18) pour aligner deux extrémités différentes de tubes coupés se faisant face tandis que l'on maintient le contact entre les extrémités de tubes coupés et l'organe de coupe (35), et on retire l'organe de coupe (35) du contact avec les tubes (16a, 18a) tout en forçant les blocs de fixation (16, 18) l'un vers l'autre, lesdites phases de passage, de déplacement et de forcement étant des mouvements orthogonaux de pivotement, et on commande le minutage dudit procédé pour obtenir automatiquement ladite liaison.

FIG. 1

# FIG. IA

# FIG. 2

FIG. 3

FIG. 4

3

0 105 587

F I G. 5A

F I G. 5B

F I G. 5C

F I G. 5D

4

F I G. 6

0 105 587

# FIG. 7

```
        ┌──────┐
        │ O    │
        │ BATT │
        └──────┘
             │
PATIENT CHECKS      ╭─────────╮
BATTERY WHICH       │  PUSH   │
RESETS MECH.        │"BATTERY"│
                    │ BUTTON  │
                    ╰─────────╯
                         │
  F I G.  8A         ◇ IS ◇  ─── YES ───┐
                     CHARGER            │
                     PLUGGED IN         │
                         │ NO           │
                    ┌──────────┐        │
                    │ TURN ON  │        │
                    │ELECTRONICS│       │
                    └──────────┘        │
                         │              │
                     ◇ IS ◇ ── YES ──┐  │
                     UNIT            │  │
                     DISABLED        │  │
                         │ NO   ┌────────┐
                              │ BEEP & │
                              │ BLINK 3│
                              │ TIMES  │
```

FIG. 8A

# FIG. 8B

```
┌──────────┐
│ O        │
│ READY    │
└──────────┘
```

```
  ╭──────────╮      PATIENT LOADS
  │  PUSH    │      TUBES THEN PUSHES
  │ "READY"  │      "READY" BUTTON
  │  BUTTON  │
  ╰──────────╯
       │
       ▼
    ◇ IS
    CHARGER ──── YES ──────────────────────┐
    PLUGGED IN                             │
       │                                   │
       NO                                  │
       ▼                                   │
  ┌──────────┐                             │
  │ TURN ON  │                             │
  │ELECTRONICS│                            │
  └──────────┘                             │
       │                                   │
       ▼                                   │
    ◇ IS                                   │
    UNIT ──── YES ────────────┐            │
    DISABLED                  │            │
       │                      │            │
       NO                     ▼            │
       ▼                 ┌──────────┐      │
    ◇ ARE               │  BEEP &   │      │
    CLAMPS              │  BLINK 3  │      │
    CLOSED & ── NO ──┐  │  TIMES    │      │
    RESET            │  └──────────┘      │
       │             │       │            │
       YES           ▼       │            │
       │         ┌──────┐    │            │
       ▼         │ BEEP │    │            │
    ◇ IS         └──────┘    │            │
    MAGAZINE ── NO ──┘       │            │
    IN POSITION              │            │
       │                     ▼            ▼
       YES              ┌──────────┐
       ▼                │ TURN OFF │
  ┌──────────┐          └──────────┘
  │ TURN ON  │
  │ "READY" LIGHT│
  └──────────┘
       │
       ▼
      (A)
```

FIG. 8C

# F I G. 8D

BEEP